(19) Europäisches Patentamt
European Patent Office
Office européen des brevets

(11) **EP 4 445 830 A1**

(12) **EUROPEAN PATENT APPLICATION**

(43) Date of publication:
**16.10.2024 Bulletin 2024/42**

(21) Application number: **23167305.4**

(22) Date of filing: **11.04.2023**

(51) International Patent Classification (IPC):
***A61B 5/00*** (2006.01)          *A61B 18/20* (2006.01)

(52) Cooperative Patent Classification (CPC):
**A61B 5/441; A61B 5/0077; A61B 5/4836;**
A61B 5/7264; A61B 18/203; A61B 2018/00684;
A61B 2018/00785; A61B 2018/1807;
A61B 2560/0233; A61B 2560/0247

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **BOURQUIN, Yannyk Parulian Julian
5656AG Eindhoven (NL)**

• **VERHAGEN, Rieko
5656AG Eindhoven (NL)**
• **DAMODARAN, Mathivanan
5656AG Eindhoven (NL)**
• **PALERO, Jonathan Alambra
5656AG Eindhoven (NL)**
• **VAN ABEELEN, Frank Anton
5656AG Eindhoven (NL)**

(74) Representative: **Philips Intellectual Property &
Standards
High Tech Campus 52
5656 AG Eindhoven (NL)**

(54) **OPTICAL SENSING SYSTEM FOR A BODY TREATMENT DEVICE**

(57)     A means for obtaining a measure directly or indirectly indicative of skin pigmentation within the context of a body-applied care device such as a body treatment device. An optical set-up comprises a camera for capturing a field of view which includes a skin patch of the user, and which also includes a reference object. A pre-defined light reflection characteristic is measured for both the skin patch and the reference object using one or more images obtained using the camera. The images may be pre-processed before detecting the light reflection characteristic to remove or compensate an ambient light contribution, to better standardize the measurement. A normalized light reflection characteristic value is determined based on normalizing the value measured for the skin to the value measured for the reference object.

FIG. 3

EP 4 445 830 A1

## Description

FIELD OF THE INVENTION

The present invention relates to the field of body treatment devices, particularly personal care devices such as hair removal devices.

BACKGROUND OF THE INVENTION

Within the field of body treatment devices, e.g. skin-applied body treatment devices, it may be advantageous to adjust settings of the device in dependence upon characteristics of the tissue surface to which it is being applied, for example in dependence upon characteristics of a skin surface to which it is being applied.

One example where this may be useful is in the field of Intense Pulsed Light (IPL) photoepilator devices, where, for safety reasons, the maximum treatment light intensity level should be set differently depending upon skin pigmentation or color.

This is addressed in at least one known device by using a skin tone sensor configured to automatically detect a skin tone using application of light in two distinct frequency bands: red and infrared. An optical detector is used to detect the light reflected in each band. This solution is limited in its applicability and its reliability. For example, ambient light can interfere with measurements and the resolution level of the measurement is limited.

SUMMARY OF THE INVENTION

The inventors associated with the present application have explored the alternative possibility of using a camera to measure characteristics of a tissue surface. A camera may enable more reliable and more precise measurements.

A camera also allows for a degree of spatial resolution in the measurement, since distribution of pigmentation over a skin patch area can potentially be detected.

However, in exploring the possibility of using a camera-based approach, the inventors have identified one or more challenges in obtaining reliable and accurate measurements that are robust to variations in ambient light conditions. Particularly in the case of home use devices, ambient light conditions can vary widely, in both spectrum and intensity. Therefore, measurement algorithms which assume fixed incident light characteristics may produce inaccurate results, with little or no control over the image signal-to-noise ratio (SNR).

To improve calibration and SNR in the captured images, the inventors considered a solution in which ambient light is removed by shielding the area to be imaged, and implementing a controlled light source to illuminate the object. However, a disadvantage of shielding the entire image area from ambient light is that the shielding typically severely hampers the user visibility of the area under treatment and, due to its size, restricts the use of the device to relatively large and flat skin surfaces.

Another approach considered by the inventors is ambient light correction based on capturing multiple images at different applied light levels and using background subtraction and/or temporal averaging. The inventors have found that this yields significant improvements in mitigating the effects of ambient light interference.

However, the inventors have recognized that this still leaves a degree of unreliability particularly in the context of mass-manufactured devices where the manufacturing tolerances for the light intensity and color spectrum of light sources allows for too wide a degree of variation to enable high precision color measurements that rely on known color properties of the incident light. Any individual light source can also exhibit instability in color and intensity output both over short-term and lifetime operation. Also, product-to-product color consistency of both the light source and camera sensor are known sources of inaccuracy. Although these variations are significantly smaller than those introduced by variations of ambient light, the inventors have recognized that they remain too large for an accurate skin tone measurement.

The inventors have sought therefore an improved optical sensing arrangement capable of addressing one or more of these challenges.

The invention is defined by the claims.

According to examples in accordance with an aspect of the invention, there is provided an optical sensing system for a body treatment device, for sensing a measure of skin pigmentation of a tissue surface to be treated by the body treatment device, the optical sensing system comprising:

a camera for imaging a skin patch;
a controllable light source for illuminating the skin patch;
a reference object positioned in a field of view of the camera, wherein the reference object comprises a physical body having a surface visible to the camera; and
a controller adapted to:

perform an image capture operation comprising controlling the light source and the camera to acquire a set of image frames, each frame spanning said field of view containing both the skin patch and the reference object;
process the one or more image frames to obtain an ambient-light corrected image representation of each of the skin patch and the reference object;
measure a value of a pre-defined light reflection characteristic of the skin patch using the ambient-light corrected image representation of the skin patch;
measure a value of the pre-defined light reflection characteristic of the reference object using the ambient-light corrected image representation of the reference object;

normalize the value of the light reflection characteristic for the skin patch to the measured light reflection characteristic value of the reference object to thereby obtain a normalized light reflection characteristic value for the skin patch; generate a data output representative of the normalized light reflection characteristic value for the skin patch.

The inventors have recognized that to ameliorate the potential uncertainty in intensity and precise color spectrum of the incident light, a reference object may be introduced to provide a local color and intensity calibration that remains reliably constant over time. It is the recognition of the inventors that manufacturing constraints for the surface reflection characteristics of such a reference object can be made much narrower and more reliable than those of the light output characteristics of the lighting device and the measurement calibration of the camera. Thus, rather than rely on known incident light properties and known color sensitivity characteristics of the camera, a reference object is introduced, and wherein the skin reflection measurements are all normalized to the measured values for the same reflection characteristics of the reference object. Thus results in a more robust and reliable measurement across time, and across pieces in a mass-manufactured operation.

With regards to obtaining the ambient light corrected image representations, various approaches are possible, and it is not essential to the present inventive concept which is used. Both hardware and software means for reducing ambient light contributions are known in the art, and various examples will be described in more detail later.

In general terms, by 'ambient-light corrected image representation' is meant an image (representation) in which an ambient light contribution is reduced, or eliminated, compared to an original version of the image. An ambient light contribution means a light contribution from background light, i.e. light which does not originate from the controllable light source of the system.

By way of one set of examples, the image capture operation may comprise: controlling a time modulation of the light source and controlling timings of camera acquisitions to acquire the set of image frames so as to together include a respective plurality of image representations of each of the skin patch and the reference object illuminated with different levels of light from the controllable light source. Obtaining the ambient-light corrected image representation of each of the skin patch and the reference object can be achieved based on combining said image representations to reduce an ambient light contribution.

By way of one example, the set of images may together include at least one portion of an image representing each of: the skin patch illuminated by the light source, the skin patch not illuminated by the light source, the reference object illuminated by the light source, and the reference object not-illuminated by the light source; and obtain an ambient-light corrected image representation of each of the skin patch and the reference object based on combining said at least portions of the set of images to reduce an ambient light contribution. Sinusoidal light control is another known option, wherein the set of images include images illuminated with smoothly varying light levels, and wherein particular combination of the images reduces the ambient contribution. EP3869781 A1 describes one example approach for instance. WO 2021/089422 A1 describes a further example approach.

In some embodiments, the pre-defined light reflection characteristic is a single-parameter value. A single-parameter value for example refers to a numerical or categorical value associated with a single characteristic, attribute, or property of an object, system, or process. In other words, it represents a single aspect or variable of a given entity. For example, it is a value of a variable that has a single degree of freedom.

It is more usual to measure skin reflection characteristics using a multi-parameter vector, including multiple values for defining color characteristics (e.g. RGB), and at least one value defining intensity. However, the inventors have found the somewhat surprising result that a single-parameter value with a single degree of freedom can be sufficient, particularly in the context of detecting skin pigmentation. The inventors have found for example that it is possible to accurately map melanin index to a single parameter of optical appearance.

In some embodiments, the pre-defined light reflection characteristic may be a measure of lightness or brightness, i.e. a measure of lightness or brightness of an imaged surface, e.g. a surface of the skin patch and/or the reference object.

By way of example, according to some embodiments, the pre-defined light reflection characteristic may be a lightness value, $L^*$, within the CIELAB color space.

The CIELAB color space, also known as the Lab* color space, is a perceptually uniform color space developed by the International Commission on Illumination (CIE). In this color space, $L^*$ represents the lightness value, with a range from 0 (black) to 100 (white).

Lightness, $L^*$, is for example well correlated with melanin level, which is a useful measurement to obtain in the context of skin-applied devices, for example for configuring the intensity level of a photoepilation device.

$L^*$ is an example of a single-parameter value which the inventors have found to work particularly well in characterizing skin pigmentation.

However, other measured of surface lightness or brightness could also be used, such as luminance (Y) in the CIEXYZ color space, relative luminance, lightness L in HSL and HSV color spaces, and many others.

In some embodiments, the controller is further adapted to convert the normalized light reflection characteristic value to a skin pigmentation value using a pre-determined lookup table.

The phrase skin pigmentation may include for example melanin index, a skin color and/or a skin type. Skin type may refer for example to a skin type classification, for example within the Fitzpatrick skin type scale.

In some embodiments, the controller is further configured to determine an operational parameter setting for the body-treatment device based on the normalized value of the light reflection characteristic of the skin patch. This may be obtained using a pre-determined look-up-table which defines mappings between different possible values for the normalized light reflection characteristic and defined settings for the operational parameter.

Another aspect of the invention is a body treatment or body care device which comprises a body-treatment subsystem for performing a body treatment function, and further comprises an optical sensing system in accordance with any embodiment described in this document.

In some embodiments, the optical sensing system is integrated in the body treatment or care device. An alternative possibility is that the optical sensing system is integrated in separate device to the body treatment or care device. The separate device may for example be configured as an attachment to the body treatment or care device or be designed to mechanically couple or mount thereto.

In some embodiments, the body-treatment subsystem comprises an operative area at a face of the body treatment device, for engagement with skin during use, and adapted to perform the body treatment function to a patch of skin aligned with the operative area. The operative area may be adjacent the field of view of the camera of the optical sensing system. The operative area may be non-overlapping with the field of view of the camera of the optical sensing system. The operative area may be contiguously adjacent to the field of view of the camera is some embodiments.

In some embodiments, the controller of the optical sensing system may be configured to: determine an operational parameter setting for the body-treatment sub-system based on the normalized value of the light reflection characteristic of the skin patch, using a pre-determined look-up-table which defines mappings between different possible values for the normalized light reflection characteristic and defined settings for the operational parameter; and generate a control signal for configuring the operational parameter setting of the body treatment subsystem.

In some embodiments, the body treatment device is a photoepilation device. The body-treatment subsystem may be a photoepilation sub-system comprising: a treatment light source for applying treatment light to a skin region; and a treatment driving unit for driving the treatment light source.

The treatment driving unit may be controlled by a controller. For example, this may be the same controller as is used to control the image capture and the determination of the light reflection characteristic. Alternatively, a dedicated controller may be provided.

Another aspect of the invention is a method for use during operation of a body treatment device for sensing a measure of skin pigmentation of a tissue surface to be treated by the body treatment device. The method comprises: obtaining a set of image frames using a camera and a controllable light source, each image frame spanning a field of view which includes a skin patch and includes a fixed reference object, the reference object comprising a physical body having a surface visible to the camera; generating an ambient-light corrected image representation of each of the skin patch and the reference object based on processing of the one or more image frames; measuring a value of a pre-defined light reflection characteristic of the skin patch using the ambient-light corrected image representation of the skin patch; measuring a value of the pre-defined light reflection characteristic of the reference object using the ambient-light corrected image representation of the reference object; normalizing of the light reflection characteristic value for the skin patch to the measured light reflection characteristic value of the reference object to thereby obtain a normalized light reflection characteristic value for the skin patch; and generating a data output representative of the normalized light reflection characteristic value for the skin patch.

In some embodiments, the image capture operation comprises: controlling a time modulation of the light source and controlling timings of camera acquisitions to acquire the set of image frames so as to together include a respective plurality of image representations of each of the skin patch and the reference object illuminated with different levels of light from the controllable light source.

In some embodiments, the pre-defined light reflection characteristic is a measure of a surface lightness or brightness, for example a lightness value, $L*$, within CIELAB color space.

In some embodiments, the method further comprises determining an operational parameter setting for the body-treatment device based on the normalized value of the light reflection characteristic of the skin patch, using a pre-determined look-up-table which defines mappings between different possible values for the normalized light reflection characteristic and defined settings for the operational parameter.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 outlines steps of an example method in accordance with one or more embodiments of the invention;

Fig. 2 is a block diagram of an example optical sensing system in accordance with one or more embodiments of the invention;

Fig. 3 illustrates an example body treatment device comprising an integrated optical sensing system in accordance with one or more embodiments of the invention;

Fig. 4 illustrates a relationship between lightness value, L*, and melanin index, M;

Fig. 5 illustrates error bars for skin light reflection characteristic measurements obtained without ambient light correction, not in accordance with the invention;

Fig. 6 illustrates error bars for skin light reflection characteristic measurements obtained with ambient light correction, not in accordance with the invention; and

Fig. 7 illustrates error bars for skin light reflection characteristic measurements obtained with ambient light correction and also with normalization to a measurement obtained for a reference object, in accordance with embodiments of the present invention.

## DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Embodiments of the present invention provide an improved means for obtaining a measure directly or indirectly indicative of skin pigmentation within the context of a body-applied care device such as a body treatment device. Proposed is an optical set-up which comprises a camera for capturing a field of view which includes a skin patch of the user, and which also includes a reference object. A pre-defined light reflection characteristic is measured for both the skin patch and the reference object using one or more images obtained using the camera. The images may be pre-processed before detecting the light reflection characteristic to remove or compensate an ambient light contribution, to better standardize the measurement. There is then defined a normalized light reflection characteristic value based on normalizing the value measured for the skin to the value measured for the reference object. Since reflection characteristics of the reference object are static over time, this provides for a reliable, standardized measure of skin reflection properties. This can optionally then be used to configure an operational setting for the body-applied device, for example using a pre-determined lookup table which defined mappings between measured values for the normalized reflection characteristic and operational parameter settings.

At least a subset of embodiments enables adaptation of a treatment function, e.g. intense pulsed light (IPL) treatment, based on an indirect estimation of skin pigmentation using a measured skin lightness value obtained from the combination of a camera, a light source (optionally a modulated light source) and a color calibration reference object or element.

At least a subset of embodiments of the present invention aim to provide systems and methods that provide color calibration without requiring additional calibration steps for the user and which do not further obstruct the visibility of the area under treatment and that do not rely on mechanically shielding the image area from ambient light.

It is an objective of at least a subset of embodiments to provide systems and methods that yield accurate and precise skin pigmentation or color measurements.

It is another objective of at least a subset of embodiments of the present invention to provide systems and methods that provide improved efficacy and safety of IPL home treatment achieved by adapting the treatment to the skin pigmentation or color using a camera as a sensor.

It is another objective of at least a subset of embodiments of the present invention to provide systems and methods that provide color calibration that do not require extra steps for the user and that do not further obstruct the visibility of the area under treatment and that do not rely on mechanically shielding the image area from ambient light.

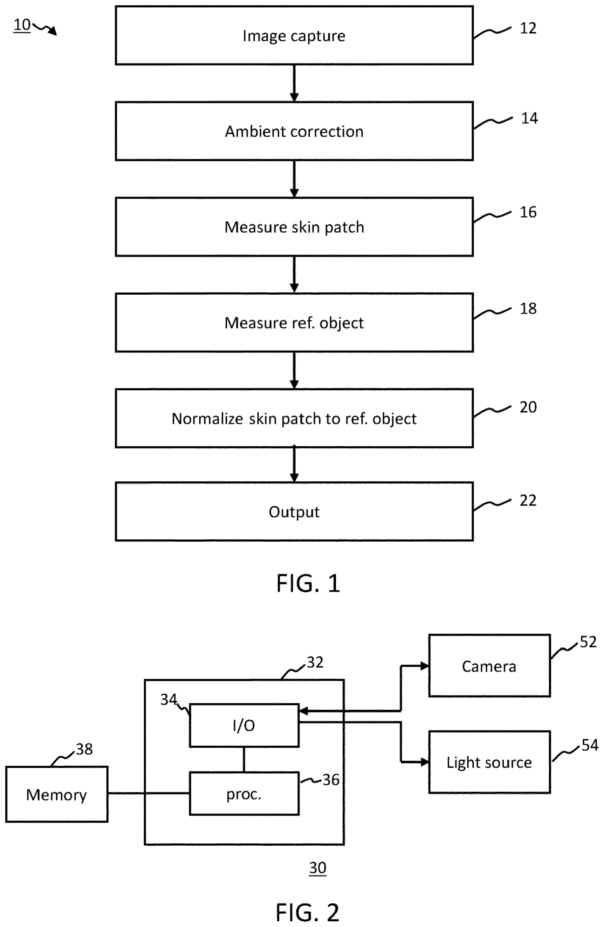

Fig. 1 outlines in block diagram form steps of an example method according to one or more embodiments. The steps will be recited in summary, before being explained further in the form of example embodiments.

Provided is a method 10 for use, for example during operation of a body treatment device, for sensing a measure of skin pigmentation of a tissue surface to be treated by the body treatment device.

The method 10 comprises obtaining 12 a set of image frames using a camera and a controllable light source, each image frame spanning a field of view which includes a skin patch and includes a fixed reference object, the reference object comprising a physical body having a surface visible to the camera. For example, the method controlling a camera and a controllable light source to obtain the set of image frames.

The method 10 further comprises generating 14 an ambient-light corrected image representation of each of the skin patch and the reference object based on processing of the one or more image frames.

The method 10 further comprises measuring 16 a value of a pre-defined light reflection characteristic of the skin patch using the ambient-light corrected image representation of the skin patch.

The method 10 further comprises measuring 18 a value of the pre-defined light reflection characteristic of the reference object using the ambient-light corrected image representation of the reference object.

The method 10 further comprises normalizing 20 of the light reflection characteristic value for the skin patch to the measured light reflection characteristic value of the reference object to thereby obtain a normalized light reflection characteristic value for the skin patch.

The method 10 further comprises generating 22 a data output representative of the normalized light reflection characteristic value for the skin patch.

As noted above, the method can also be embodied in hardware form, for example in the form of a processing device which is configured to carry out a method in accordance with any example or embodiment described in this document, or in accordance with any claim of this application.

To further aid understanding, Fig. 2 presents a schematic representation of an example controller 32 configured to execute a method in accordance with one or more embodiments of the invention. The controller is shown in the context of a system 30 which comprises the controller. The controller alone represents an aspect of the invention. The system 30 is another aspect of the invention. The provided system need not comprise all the illustrated hardware components; it may comprise only a subset of them.

The controller 32 comprises one or more processors 36 configured to perform a method in accordance with that outlined above, or in accordance with any embodiment described in this document or any claim of this application. In the illustrated example, the controller further comprises an input/output 34 or communication interface.

In the illustrated example of Fig. 2, the system 30 further comprises a camera 52 for imaging a skin patch.

In the illustrated example of Fig. 2, the system further comprises a controllable light source 54 for illuminating the skin patch.

The system may further comprise the aforementioned reference object positioned in a field of view of the camera, wherein the reference object comprises a physical body having a surface visible to the camera.

The system 30 may further comprise a memory 38 for storing computer program code (i.e. computer-executable code) which is configured for causing the one or more processors 36 of the controller 32 to perform the method as outlined above, or in accordance with any embodiment described in this disclosure, or in accordance with any claim. The memory may additionally or alternatively store one or more lookup tables, for example a pre-determined lookup table which defined mappings between measured values for the normalized reflection characteristic and operational parameter settings, and/or a lookup table defining mappings between skin lightness values, L*, and corresponding skin pigmentation values, e.g. Melanin index.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application. For example, the computer program code may be configured, when run on a processor, to cause the processor to:

> receive a set of image frames, each image frame spanning a field of view which includes a skin patch and includes a fixed reference object, the reference object comprising a physical body having a surface visible to the camera;
> generate an ambient-light corrected image representation of each of the skin patch and the reference object based on processing of the one or more image frames;
> measure a value of a pre-defined light reflection characteristic of the skin patch using the ambient-light corrected image of the skin patch;
> measure a value of the pre-defined light reflection characteristic of the reference object using the ambient-light corrected image of the reference object;
> normalize of the light reflection characteristic value for the skin patch to the measured light reflection characteristic value of the reference object to thereby obtain a normalized light reflection characteristic value for the skin patch;
> generate a data output representative of the normalized light reflection characteristic value for the skin patch.

The computer program code may be configured, when run on a processor, to cause the processor to control the camera and the light source to obtain the set of image frames.

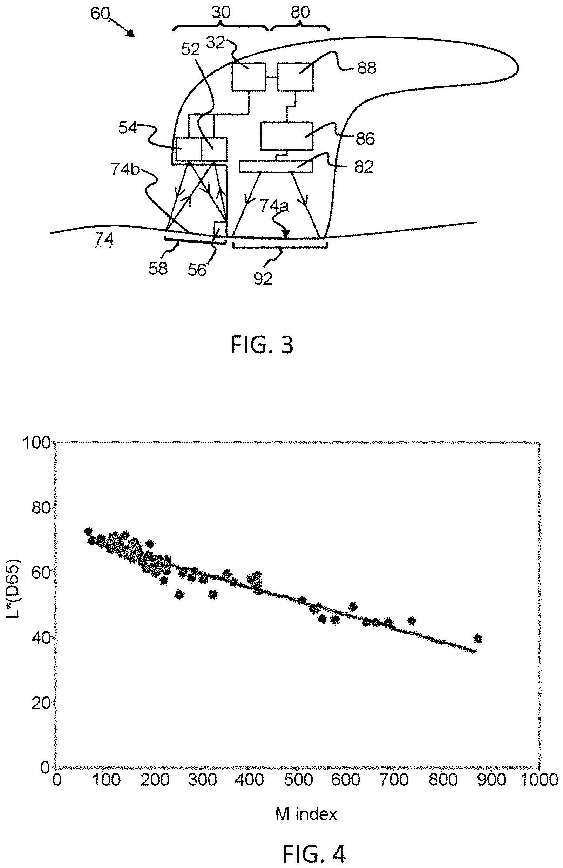

Fig. 3 schematically illustrates an example body treatment device incorporating an optical sensing system according to one or more embodiments of the invention.

The device comprises an optical sensing system 30. The device further comprises a body-treatment subsystem 80 for performing a body treatment function.

The optical sensing system 30 comprises a camera 52 for imaging a skin patch 74b. The camera has an imaging field of view 58. The optical sensing system further comprises a controllable light source 54 for illuminating the skin patch 74b. The optical sensing system further comprises a reference object 56 positioned the field of view 58 of the camera, wherein the reference object comprises a physical body having a surface visible to the camera 52. The reference object may be arranged to make contact with the surface of the skin. The optical sensing system further comprises a controller 32 for controlling the camera and the controllable light source. The controller is adapted to control implementation of the steps of a method discussed previously.

For example, the controller 32 is adapted to: perform an image capture operation comprising controlling the light source 54 and the camera 52 to acquire a set of image frames, each frame spanning said field of view containing both the skin patch 74b and the reference object 56. The light source generates a light output which has an area of incidence which overlaps with the field of view of the camera. For example, it spans at least a portion of the field of view 58 of the camera. For example, it generates a light output which fully spans the field of view of the camera. The light source 54 may for example comprise one or more LEDs. As will be explained later, in some embodiments, the light source 54 may be controlled to provide a modulated illumination, meaning that an intensity of the illumination is varied as a function of time, for example either in a pulsed control scheme or a continuous variation scheme, e.g. sinusoidal variation. However, this form of control is not essential.

With regards to the light source 54, the specific spectral characteristics are not critical to the functionality of the system. In some embodiments, the light source spectrum may have a single or multiple narrow band(s), either generated by a single lighting element (e.g. LED) or a plurality of lighting elements. In other embodiments, the light source spectrum may have a wide band. For example, suitable arrangements may include an LED with a phosphor light conversion layer, a gas-discharge lamp, or an incandescent lamp. As will be explained later, in some embodiments, the obtained light reflection characteristic is lightness, $L^*$. In this case, an advantageous choice for the light source is a wide-band spectrum with a white appearance. For such a source, $L^*$ is particularly well correlated with melanin index, as is discussed later in this disclosure.

With regards to the camera 52, a variety of suitable devices exist in the art. In some embodiments, the camera may be a standard RGB camera which generates 2D images, wherein each pixel is encoded in the RGB color space.

In accordance with further embodiments, a black-and-white or monochrome camera may be used. In some embodiments, the monochrome camera may comprise a filter, e.g. a Y filter with a transmission proportional to the CIEXYZ $\bar{y}(\lambda)$ color matching function. The CIEXYZ $\bar{y}(\lambda)$ color matching function is a standard used to quantity the response of human vision to different colors of light. A Y filter of this kind filters light such that an amount of light passed by the filter is proportional to the brightness of the color as perceived by the human eye. The result is an image that has a tonal range similar to that which would be perceived by a human observer.

As will be explained later, in accordance with some embodiments, a rolling shutter camera may be used. However, in accordance with other embodiments, a global shutter camera can be used.

The controller 32 is further adapted to process the one or more image frames to obtain an ambient-light corrected image representation of each of the skin patch 74b and the reference object 56; measure a value of a pre-defined light reflection characteristic of the skin patch using the ambient-light corrected image representation of the skin patch; measure a value of the pre-defined light reflection characteristic of the reference object using the ambient-light corrected image of the reference object; normalize the value of the light reflection characteristic for the skin patch to the measured light reflection characteristic value of the reference object to thereby obtain a normalized light reflection characteristic value for the skin patch; generate a data output representative of the normalized light reflection characteristic value for the skin patch.

For the purposes of the illustrated example of Fig. 3 the body treatment device is a photoepilation device. The body-treatment sub-system 80 in this case is a photoepilation sub-system. The body-treatment sub-system 80 comprises a treatment light source 82 for applying treatment light to a skin region 74a. The body treatment sub-system 80 further comprises a treatment driving unit 86 for driving the treatment light source. For example the treatment driving unit 86 may define one or more driving parameters, e.g., voltage, pulse duration, etc. The treatment driving unit may be controlled by a controller. For example this may be the same controller 32 as is used to control the image capture and the determination of the light reflection characteristic. Alternatively, and as illustrated in Fig. 3, a dedicated controller 88 may be provided.

As illustrated, the body-treatment subsystem 80 comprises an operative area 92 at a face of the body treatment device 60, for engagement (e.g. at least optical engagement, and optionally also mechanical engagement) with skin during use, and adapted to perform the body treatment function to a patch of skin 74a aligned with the operative area. The operative area 92 is adjacent the field of view 58 of the camera 52 of the optical sensing system 30. For example, it may be contiguously adjacent.

With regards to the treatment light source 82, different options are possible. For example, this may comprise a gas discharge lamp or a laser light source.

As discussed above, a part of the method comprises processing the one or more image frames to obtain an ambient-light corrected image representation of each of the skin patch and the reference object. There are a variety of different methods for achieving this which are known in the art.

According to a simplest method, a first image of the entire field of view may be acquired with the light source 54 activated and a second image of the field of view acquired with the light source 54 deactivated. The deactivated-light image (second image) effectively represents an ambient light contribution to the first image. By subtracting the second image from the first image, a residual image is arrived at which approximately represents an ambient-free image. This may then be used as an ambient- light corrected image. The region of this image which spans the skin patch 74b can be used as the ambient-light corrected image representation of the skin patch, and the region of the image which spans the reference object 56 can be used as the ambient-light corrected image representation of the reference object.

While this simple approach is acceptable, more sophisticated methods of obtaining ambient light corrected images may produce more consistent and robust results.

One approach which may be applied is described in detail in the document WO 2021/089422 A1. This describes a system and method for performing ambient light correction in an image. The system has a light source that provides pulsed illumination to an object and a rolling shutter imaging unit that captures a series of images of the object while it is illuminated. The captured images have bright bands and dark bands, which correspond to high and low states of the pulsed illumination, respectively. The system has a control unit that generates two composite images by combining sections from the captured images that correspond to bright and dark bands. The control unit then generates an ambient light corrected image based on the difference between the two composite images. In more detail, the modulation frequency and camera line integration time are such that each frame captured by the camera will contain a structure of bright and dark bands, whereby sequential images contain the same band structure, phase shifted by a certain amount, such that the mathematical combination of these images leads to either a foreground illumination or an ambient illumination image.

Although the document describes application of this method in particular for a digital mirror device, it can be applied without major alteration to the system of embodiments of the present invention to obtain the ambient light corrected image representations. In particular, the controllable light source 54 can be controlled by the controller 32 to perform the light modulation pattern detailed in WO 2021/089422 A1, and the camera 52 can be controlled with the roller shutter imaging approach described in order to acquire the described series of images. The controller 32 can be used to generate the composite images and to generate the ambient light corrected image based on the composite images.

For implementing the method of WO 2021/089422 A1, the camera 52 may be provided as a roller shutter camera, or at least have functionality permitting operation in roller shutter mode. A rolling shutter captures an image by scanning the scene row-by-row or line-by-line (or multiple rows or lines at a time). Consequently, different parts of the image are exposed to light at slightly different moments in time.

Alternatively, if a global shutter camera is to be used, the simpler method described previously may be used, wherein the camera 52 acquisition timings are synchronized with the illumination source 54 in order to obtain images with the light on and with the light off, and the off image is subtracted from the on image to obtain the ambient light corrected image.

A further approach which may be applied is described in detail in the document EP3869781 A1. This describes acquiring a sequence of images while implementing a sinusoidal illumination control. Using the sinusoidal intensity modulation in time, the imaging device is configured such that a different spatial intensity modulation pattern is provided in consecutive respective images of the sequence. The method determines an estimated intensity distribution for an image of the subject based on the spatial intensity modulation pattern in each of the sequence of images, to reduce ambient lighting variation in the image.

As a more general principle of implementation, the obtaining of the ambient light corrected images may involve the following in terms of the image capture operation: controlling a time modulation of the light source 54 and controlling timings of camera acquisitions to acquire the set of image frames so as to together include a respective plurality of image representations of each of the skin patch 74b and the reference object 56 illuminated with different levels of light from the controllable light source 54; and obtaining the ambient-light corrected image representation of each of the skin patch and the reference object based on combining said image representations to reduce an ambient light contribution.

In some embodiments, the set of images may together include at least one portion of an image representing each of: the skin patch illuminated by the light source, the skin patch not illuminated by the light source, the reference object illuminated by the light source, and the reference object not-illuminated by the light source. The ambient-light corrected image representation of each of the skin patch and the reference object may be obtained based on combining said at least portions of the set of images to reduce an ambient light contribution. However, as discussed above, sinusoidal or other continuous variation light modulation controls may also be considered.

The ambient light correction described above goes some way toward ameliorating unreliability in measurements, as far as these are caused by ambient light variation, but does not fully solve the problem. This is because light sources are known to be unstable in both their intensity and color/emission spectrum over both short-term and lifetime operation. Also, product-to-product color consistency of both the light source and camera sensor are well known sources of inaccuracy, and although these variations are significantly smaller than those introduced by the variations of the ambient, they are still larger than would be ideal for obtaining accurate skin tone measurement.

Accordingly, the normalization step is introduced, as discussed above, wherein the light reflection characteristic measured for the skin patch 74b is normalized to the light reflection characteristic value measured for the reference objection 56.

With regards to the normalization, this may comprise simply dividing the value of the light reflection characteristic for the skin patch by the measured light reflection characteristic value of the reference object to thereby obtain the normalized light reflection characteristic value for the skin patch. Optionally, the result may be further multiplied by a multiplier coefficient, $\alpha$, to bring the value into a specific desired range.

For example, a normalized value of the light reflection characteristic, $A_{norm}$, may be obtained according to:

$$A_{norm} = \alpha\,(A\,/\,B)$$

where A is the measured value of the light reflection characteristic for the skin patch, B is the measured value of the light reflection characteristic for the reference object, and $\alpha$ is a multiplier to set the value range. If desired, $\alpha$ may simply be set to 1.

With regards to the light reflection characteristic which is measured, this is preferably a single-parameter value.

For example, in some embodiments, the measured light reflection characteristic may be a measure of lightness or brightness of a surface.

By way of one advantageous example, the measured light reflection characteristic may be a lightness value, L*, within CIELAB color space.

The CIELAB color space, also known as the Lab* color space, is a perceptually uniform color space developed by the International Commission on Illumination (CIE). In this color space, L* represents the lightness value, with a range from 0 (black) to 100 (white).

As mentioned above, in some embodiments, the camera is a monochrome camera with a Y filter, wherein the transmission of the filter is proportional to the CIEXYZ $\bar{y}(\lambda)$ color matching function. A monochrome camera is a camera that captures images in grayscale, registering only the intensity of light at each pixel.

To obtain an L* value from an image captured with such a camera, the process is straightforward. Following the definition of CIELAB color space, a value for L* can be obtained as follows:

$$L^* = 116\,f(A\_norm) - 16\,,$$

where L* represents the lightness value in the CIELAB color space, A_norm is the normalized luminance value calculated from the captured image (which is obtained by dividing the actual luminance (Y) obtained from the image by the reference white luminance (Yn)), and where $f(t) = t^{1/3}$ for t > 0.0088.

With regards to the actual luminance value, Y, when using a monochrome camera and Y filter, each pixel in the image has a respective luminance value, and these values can be used to calculate an overall luminance of the image or of target regions of interest within the image (e.g. an area spanning the skin region and an area spanning the reference object). For example an average luminance can be taken over a relevant area or another calculation approach can be used.

The reference white luminance, Yn, is the luminance value of a standard white reference. Common white references include the CIE standard illuminants, such as D65 or D50.

CIELAB also gives a definition of f(t) for smaller values of t but that is not relevant in this context.

To obtain an L* value from a color (e.g. RGB )camera image, it will typically be necessary to convert the color values encoded in pixels of the image into the CIELAB color space. One approach to doing this is through a two-step conversion; first converting color values from the RGB image into the CIEXYZ color space, and then converting from the CIEXYZ color space into the CIELAB color space.

The conversion formulas for the first step can vary based on the specific RGB color space being used (e.g., sRGB, Adobe RGB). For the sRGB color space, the conversion can be performed using gamma correction, followed by a linear transformation. The pixel color values in the CIEXYZ space may then be converted into the CIELAB space. The L* value for each pixel in the image can then be extracted.

The conversion formulas from sRGB and CIEXYZ are well known and the conversion formulas from CIEXYZ to CIELAB are also well known. See e.g.

- Wikipedia. (2023, March 24). SRGB. In Wikipedia, The Free Encyclopedia. Retrieved 10:49, March 29, 2023, from https://en.wikipedia.org/w/index.php?title=SRGB&oldid=1146324346, and
- Wikipedia. (2023, January 4). CIELAB color space. In Wikipedia, The Free Encyclopedia. Retrieved 10:50, March 29, 2023, from https://en.wikipedia.org/w/index.php?title=CIELAB_color_space&oldid=1131550422

This results in a measure of the lightness value, L*, within the CIELAB color space, for each pixel of the camera image.

To obtain the measured light reflection characteristic value, the L* values for the image may be further processed. For example, the values over a whole region of the image spanning each of the skin patch and the reference object could be respectively averaged to obtain single measurements for L* for each of the skin patch and the reference object. Alternatively a single representative L* value could be selected from among the pixels contained in each of the aforementioned regions, for example a maximum or minimum value. In other words, as a more general principle, the method may comprise extracting an L* value based on the L* values of pixels spanning the skin patch; and extracting an L* value based on the L* values of pixels spanning the reference object. These may be an average value of pixels spanning the skin patch for example and/or average value of pixels spanning the reference object for example.

L* is suggested as a particularly advantageous characteristic to utilize in accordance with one or more embodiments of the invention because it correlates well with Melanin index, which is a useful measurement to obtain in the context of skin-applied devices, for example for configuring the intensity level of a photoepilation device. In particular, melanin index provides a useful proxy for skin pigmentation. Melanin index is a measure of the amount of melanin in a person's skin.

In other words, by measuring a light reflection characteristic of L*, this provides a proxy measure of skin pigmentation, by virtue of the strong correlation between L* and melanin index.

This correlation has been demonstrated in data from several experimental studies. Fig. 4 illustrates a plot of lightness L* (y-axis) against melanin index, MI, (x-axis) and shows that the two are very well correlated. The R-squared, R-sq, is 90.8% and the Adjusted R-squared, R-Sq(adj) is 90.7%.

Optionally, the measured value of L* could be converted into a measure of melanin index via the known correlation. For example, in the result presented in Fig. 4, the mapping between L* and Melanin Index is as follows: L* = 72.33 - 0.04223 M, where M is melanin index, and L* is lightness value.

Although L* is suggested as one example of a single-parameter value for characterizing the optical appearance of the skin patch and the reference object, there are numerous other single-parameter values that could additionally or alternatively be employed to characterize the optical appearance of a surface, and in particular, the lightness or brightness . Examples include:

- luminance (Y) in the CIEXYZ color space, which corresponds to perceived brightness.
- Relative luminance.
- Gamma-corrected luminance.
- Lightness L in HSL and HSV color spaces: The Lightness (L) component in HSL and the Value (V) component in HSV provide respectively representations of lightness or brightness of a surface.
- Luma Y' in Y'CbCr and Y'UV color spaces.
- Chroma (C*) in CIELCH color space which is a measure of color saturation relative to brightness.
- Whiteness index (WI).

- Gloss, which is a measure of specular reflectance of a surface.
- Nits (candela per square meter).

All of these characteristics represent a respective measure of the lightness or brightness of a surface.

In some embodiments, the obtained normalized light reflection characteristic may be converted to a skin pigmentation value using a pre-determined lookup table.

In some embodiments for example, the obtained light reflection characteristic may be converted to a value of Melanin Index using a pre-determined relationship or mapping (see above discussion with reference to Fig. 4).

In some embodiments, the obtained light reflection characteristics may be converted to a skin type classification, for example within the Fitzpatrick skin type scale. Different sub-ranges of the light reflection characteristic, e.g. Lightness L*, may be defined as corresponding to different classifications within such a skin type classification system.

As mentioned previously, in some embodiments, the controller is further adapted to configure an operational parameter setting of the body treatment device in dependence upon the normalized value of the light reflection characteristic of the skin patch.

By way of example, this feature may be implemented using a pre-determined look-up-table which defines mappings between different possible values for the normalized light reflection characteristic and defined settings for the operational parameter.

By way of example, a lightness value, L*, spans from 0 to 100. A lookup table may be defined in advance which maps different ranges of values of the lightness, L*, to different operational parameter settings. For example, if the device is a photoepilator device, such as discussed above, the different operation parameter settings could be different intensity settings for the treatment light of the treatment light source 82.

For example, light intensity may be decreased for darker skin tone, and increased for lighter skin tone.

Thus, it is proposed in some embodiments to set a value of an operational parameter based on the obtained normalized light reflection characteristic, or based on a skin pigmentation value, skin color value or skin type classification derived from this normalized value, using a reference look-up-table (LUT). The method may further comprise setting the device to the value of the operational parameter. For example, the operational parameter setting may be a treatment light intensity value, and wherein the controller is adapted to set the treatment light based on the obtained treatment setting value and to provide the treatment light on the treatment skin patch.

In some embodiments, the measurement of the normalized light reflection characteristic for a skin region may be performed in a pre-treatment session (or 'dry run'), for example as part of an initialization or setup phase. For example, the user may apply the device over an area which is to be treated, and the image acquisition procedure is performed, ambient light correction applied, and the normalized light reflection characteristic obtained. The operational parameter setting may then be configured in dependence upon the value of the light reflection characteristic measured in the pre-treatment phase.

In some embodiments, the controller may include additional functionality for performing image analysis of at least one image acquired by the camera, for example in the image acquisition procedure or otherwise. For example, the controller may include a classification module for classifying one or more features or properties or characteristics of a skin region. The classification module may employ a machine learning algorithm, for example a trained neural network. For example, pigmented spots or skin lesions or other skin pathologies may be detected by the classification module, and wherein an operational parameter setting is adjusted in dependency thereon. Detected features and/or skin tone may be classified and checked against contraindications such that the device automatically adjusts, blocks or gives advice during the actual treatment session.

With regards to the reference object 56, the purpose of this is to provide a locally stable optical reference against which the measured light reflection characteristic for the skin patch 74b or skin region can be calibrated. It might therefore otherwise be referred to as a 'calibration element'.

As discussed above, a (preferably ambient-corrected) image representation of each of the skin patch 74b and the reference object 56 are obtained, the value of light reflection characteristic sampled or measured for each of the skin patch and the reference object, and wherein the skin patch value is normalized to the reference object value.

In this context, the specific value of the light reflection characteristic possessed by the visible surface of the reference object is not essential; its purpose is to provide a locally stable reference and for this purpose almost any specific value can be used. However, the inventors have found that a lighter surface color for the reference object may be preferable for the reason that this yields a higher signal-to-noise (SNR). In particular, for a camera, SNR typically improves towards an upper end of the dynamic range. Therefore, it is advantageous to provide a reference with a high brightness relative to the skin. The SNR in the measured reflection characteristic of the reference will then contribute less to the final output, i.e. the normalized value of the reflection characteristic of the skin.

By way of example, the surface of the reference object visible to the camera may be made a light grey color, or white color, or near white / off-white color.

In terms of materials, any convenient material may be used, including for example plastics or ceramics or a coating.

The proposed system in accordance with Fig. 2 has been tested experimentally by the inventors.

Images of skin patches were collected which included images of skin types corresponding to each of skin types 1 to 6 within the Fitzpatrick skin type scale.

In acquiring the images, the skin was deliberately subjected to ambient light of varying conditions, to mimic real life ambient light variation. The ambient light was varied both in terms of light temperature and light intensity. In addition, noise was artificially added to the images to also mimic realistic variations expected due to known inherent instability of mass-produced light sources in consumer devices. The images were obtained using a standard (RGB) camera and ambient light correct performed.

Each image also contained a reference object 56 in accordance with the discussions previously for providing a color reference.

Different levels of ambient light correction and color normalization were applied to the images, for the purposes of testing the proposed system according to embodiments of the present invention. In particular, the resulting images included:

1. Standard camera images without ambient light correction;
2. Ambient light corrected images (e.g. using the rolling shutter and light modulation technique discussed above, or any alternative technique)
3. Ambient light corrected images normalized to the reference object, in accordance with one or more embodiments of the present invention.

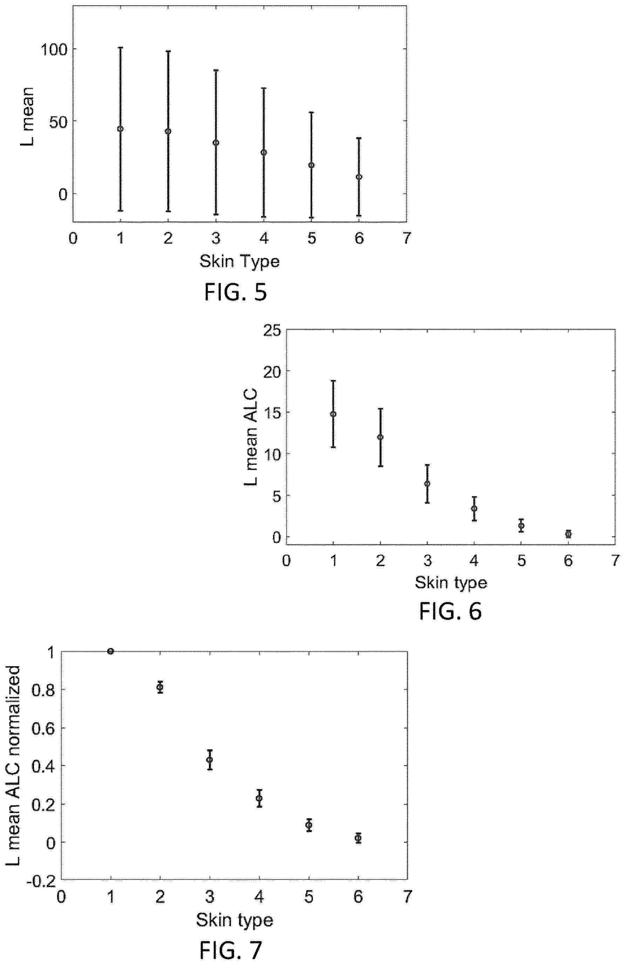

The results for each of these three image groups are shown respectively in Fig. 5, Fig. 6 and Fig. 7.

Fig. 5 shows, for each of skin types 1-6 (x-axis), a mean lightness, L*, value (y-axis) measured across a respective set of images depicting a skin patch of that type, and wherein the images correspond to group (1) above (no ambient light correction and no normalization). For each skin type, an error bar is also shown representing 3* the standard deviation in the measured L* values across the respective set of images. It may be seen that there is a large standard deviation in the L* measured across the images of each skin type. Indeed, the size of the standard deviation is such that it would be difficult or even impossible to distinguish from the L* values alone, one skin type from another.

Fig. 6 shows, for each of skin types 1-6 (x-axis), a mean lightness, L*, value (y-axis) measured across a respective set of images depicting a skin patch of that type, and wherein the images correspond to group (2) above (ambient light correction applied but no normalization). For each skin type, an error bar is also shown representing 3* the standard deviation in the measured L* values across the respective set of images. For this particular experiment, the ambient light correction was performed in accordance with the method described in WO 2021/089422 A1. It may be observed that the standard deviation is reduced compared to the images with no ambient light correction (Fig. 5), but is still higher than would be ideal in order to guarantee reliable skin type classifications.

Fig. 7 shows, for each of skin types 1-6 (x-axis), a mean lightness, L*, value (y-axis) measured across a respective set of images depicting a skin patch of that type, and wherein the images correspond to group (3) above (ambient light correction applied and color normalization according to one or more embodiments of the invention). For each skin type, an error bar is also shown representing 3* the standard deviation in the measured L* values across the respective set of images. It can be seen that the standard deviation is very greatly reduced, enabling a clear distinction to be made between different skin types based on measured Lightness value.

Embodiments of the invention described above employ a controller. The controller may comprise a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. An optical sensing system (30) for a body treatment device, for sensing a measure of skin pigmentation of a tissue surface to be treated by the body treatment device, the optical sensing system comprising:

   a camera (52) for imaging a skin patch (74b);
   a controllable light source (54) for illuminating the skin patch;
   a reference object (56) positioned in a field of view (58) of the camera, wherein the reference object comprises a physical body having a surface visible to the camera; and
   a controller (32) adapted to:

      perform (12) an image capture operation comprising controlling the light source and the camera to acquire a set of image frames, each frame spanning said field of view containing both the skin patch and the reference object;
      process (14) the one or more image frames to obtain an ambient-light corrected image representation of each of the skin patch and the reference object;
      measure (16) a value of a pre-defined light reflection characteristic of the skin patch using the ambient-light corrected image representation of the skin patch;
      measure (18) a value of the pre-defined light reflection characteristic of the reference object using the ambient-light corrected image representation of the reference object; normalize (20) the value of the light reflection characteristic for the skin patch to the measured light reflection characteristic value of the reference object to thereby obtain a normalized light reflection characteristic value for the skin patch; generate (22) a data output representative of the normalized light reflection characteristic value for the skin patch.

2. The system of claim 1, wherein the image capture operation comprises:

controlling a time modulation of the light source and controlling timings of camera acquisitions to acquire the set of image frames so as to together include a respective plurality of image representations of each of the skin patch and the reference object illuminated with different levels of light from the controllable light source; and obtaining the ambient-light corrected image representation of each of the skin patch and the reference object based on combining said image representations to reduce an ambient light contribution.

3. The system of claim 1, wherein the pre-defined light reflection characteristic is a single-parameter value.

4. The system of claim 3, wherein the pre-defined light reflection characteristic is a measure of surface lightness or brightness.

5. The system of claim 4, wherein the pre-defined light reflection characteristic is a lightness value, L*, within CIELAB color space.

6. The system of any claims 1-5, wherein the controller is further adapted to convert the normalized light reflection characteristic to a skin pigmentation value using a pre-determined lookup table.

7. The system of any of claims 1-6, wherein the controller is further configured to determine an operational parameter setting for the body-treatment device based on the normalized value of the light reflection characteristic of the skin patch, using a pre-determined look-up-table which defines mappings between different possible values for the normalized light reflection characteristic and defined settings for the operational parameter.

8. A body treatment device, comprising:

the optical sensing system of any preceding claim; and a body-treatment subsystem for performing a body treatment function.

9. The device of claim 8, wherein the body-treatment subsystem comprises an operative area at a face of the body treatment device, for engagement with skin during use, and adapted to perform the body treatment function to a patch of skin aligned with the operative area, and wherein the operative area is adjacent the field of view of the camera of the optical sensing system.

10. The device of claim 8 or 9, wherein the controller of the optical sensing system is configured to:

determine an operational parameter setting for the body-treatment sub-system based on the normalized value of the light reflection characteristic of the skin patch, using a pre-determined look-up-table which defines mappings between different possible values for the normalized light reflection characteristic and defined settings for the operational parameter; and generate a control signal for configuring the operational parameter setting of the body treatment subsystem.

11. The device of any of claims 8-10, wherein the body treatment device is a photoepilation device, and wherein the body-treatment sub-system is a photoepilation sub-system comprising:

a treatment light source for applying treatment light to a skin region; and a treatment driving unit for driving the treatment light source,

12. A method for use during operation of a body treatment device, for sensing a measure of skin pigmentation of a tissue surface to be treated by the body treatment device, the method comprising:

obtaining (12) a set of image frames using a camera and a controllable light source, each image frame spanning a field of view which includes a skin patch and includes a fixed reference object, the reference object comprising a physical body having a surface visible to the camera; generating (14) an ambient-light corrected image representation of each of the skin patch and the reference object based on processing of the one or more image frames; measuring (16) a value of a pre-defined light reflection characteristic of the skin patch using the ambient-light corrected image of the skin patch; measuring (18) a value of the pre-defined light reflection characteristic of the reference object using the ambient-light corrected image of the reference object;

normalizing (20) of the light reflection characteristic value for the skin patch to the measured light reflection characteristic value of the reference object to thereby obtain a normalized light reflection characteristic value for the skin patch;

generating (22) a data output representative of the normalized light reflection characteristic value for the skin patch.

13. The method of claim 12, wherein the image capture operation comprises

controlling a time modulation of the light source and controlling timings of camera acquisitions to acquire the set of image frames so as to together include a respective plurality of image representations of each of the skin patch and the reference object illuminated with different levels of light from the controllable light source.

14. The method of claim 12 or 13, wherein the pre-defined light reflection characteristic is a measure of a lightness or brightness of an imaged surface, for example a lightness value, L*, within CIELAB color space.

15. The method of any of claims 12-14, further comprising determining an operational parameter setting for the body-treatment device based on the normalized value of the light reflection characteristic of the skin patch, using a pre-determined look-up-table which defines mappings between different possible values for the normalized light reflection characteristic and defined settings for the operational parameter.

10

| Image capture | 12 |

| Ambient correction | 14 |

| Measure skin patch | 16 |

| Measure ref. object | 18 |

| Normalize skin patch to ref. object | 20 |

| Output | 22 |

FIG. 1

32

34 I/O

38 Memory

proc. 36

Camera 52

Light source 54

30

FIG. 2

FIG. 3

M index

FIG. 4

FIG. 5

FIG. 6

FIG. 7

Europäisches
Patentamt

European
Patent Office

Office européen
des brevets

## EUROPEAN SEARCH REPORT

**Application Number**

EP 23 16 7305

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/030372 A1 (JUNG BYUNGJO [US] ET AL) 10 February 2005 (2005-02-10)<br>* figures 1A, 2A *<br>* paragraphs [0011] – [0013], [0020] – [0021], [0023] – [0024], [0026], [0028] * | 1-15 | INV.<br>A61B5/00<br><br>ADD.<br>A61B18/20 |
| X | EP 3 251 332 B1 (HEALTHY IO LTD [IL]) 7 July 2021 (2021-07-07)<br>* figure 2 *<br>* paragraphs [0014], [0020], [0022] – [0023], [0047] – [0048], [0053] – [0056], [0099] * | 1-15 | |
| A | KONDO TOMOYA ET AL: "A proposal of ambient light estimation methods for skin region detection",<br>2015 21ST KOREA-JAPAN JOINT WORKSHOP ON FRONTIERS OF COMPUTER VISION (FCV), IEEE, 28 January 2015 (2015-01-28), pages 1-6, XP032775547,<br>DOI: 10.1109/FCV.2015.7103706<br>[retrieved on 2015-05-07]<br>* abstract * | 1,12 | |

**TECHNICAL FIELDS SEARCHED (IPC)**

A61B

The present search report has been drawn up for all claims

| Place of search | Date of completion of the search | Examiner |
|---|---|---|
| The Hague | 27 September 2023 | Albrecht, Ronald |

EPO FORM 1503 03.82 (P04C01)

## ANNEX TO THE EUROPEAN SEARCH REPORT
## ON EUROPEAN PATENT APPLICATION NO.

EP 23 16 7305

This annex lists the patent family members relating to the patent documents cited in the above-mentioned European search report.
The members are as contained in the European Patent Office EDP file on
The European Patent Office is in no way liable for these particulars which are merely given for the purpose of information.

27-09-2023

| Patent document cited in search report | | Publication date | Patent family member(s) | | Publication date |
|---|---|---|---|---|---|
| US 2005030372 | A1 | 10-02-2005 | NONE | | |
| EP 3251332 | B1 | 07-07-2021 | EP | 3251332 A1 | 06-12-2017 |
| | | | US | 2018008188 A1 | 11-01-2018 |
| | | | US | 2019290187 A1 | 26-09-2019 |
| | | | US | 2021251563 A1 | 19-08-2021 |
| | | | US | 2023270373 A1 | 31-08-2023 |
| | | | WO | 2016120862 A1 | 04-08-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3869781 A1

- WO 2021089422 A1

**Non-patent literature cited in the description**

- SRGB. *Wikipedia, The Free Encyclopedia,* 24 March 2023, https://en.wikipedia.org/w/index.php?title=SRGB&oldid=1146324346

- CIELAB color space. *Wikipedia, The Free Encyclopedia,* 04 January 2023, https://en.wikipedia.org/w/index.php?title=CIELAB_color_space&oldid=1131550422